# FASCICULE DE BREVET EUROPEEN

(11) **EP 4 009 865 B1**
(45) Date de publication et mention de la délivrance du brevet: **06.11.2024**
(21) Numéro de dépôt: 20753937.0
(22) Date de dépôt: 07.08.2020
(51) Int. Cl.: A61B 5/145, A61B 5/1468, A61B 5/1455

(54) **SYSTEME DE SURVEILLANCE CORPORELLE COMPRENANT UNE MICROAIGUILLE**
KÖRPERÜBERWACHUNGSSYSTEM MIT EINER MIKRONADEL
BODY MONITORING SYSTEM COMPRISING A MICRONEEDLE

(30) Priorité: 08.08.2019 EP 19290067
(43) Date de publication de la demande: 15.06.2022
(73) Titulaire: WIZP AS, 3183 Horten (NO)
(72) Inventeur: PIERART, Luc, 94800 Villejuif (FR)
(74) Mandataire: Regimbeau
(86) Numéro de dépôt international: PCT/EP2020/072312
(87) Numéro de publication internationale: WO 2021/023884

(56) Documents cités:
- CN-A- 109 303 976
- US-A1- 2006 030 812
- US-A1- 2015 313 527
- US-A1- 2018 161 563
- US-A1- 2018 338 713
- US-A1- 2018 362 334
- US-A1- 2019 143 090

## Description

### DOMAINE DE L'INVENTION

La présente invention concerne un dispositif de mesure d'un analyte corporel. Plus précisément, elle concerne un dispositif de surveillance corporelle par une analyse de liquide corporel, typiquement interstitiel.

### ETAT DE LA TECHNIQUE

Certaines pathologies comme le diabète nécessitent une surveillance quotidienne de paramètres biochimiques du corps humain, en particulier des concentrations en certains composés (la glycémie dans l'exemple du glucose).

Pour cela, il est courant de piquer un point de la peau de sorte à faire perler une goutte de sang, et d'analyser cette goutte soit de façon réactive (par exemple avec une bandelette), soit de façon électronique (par exemple par au moins un capteur analytique), de façon à estimer le ou les paramètres cibles.

On connaît aujourd'hui des systèmes évolués bien moins invasifs qui se contentent d'analyser le liquide interstitiel, c'est-à-dire le fluide qui remplit l'espace entre les capillaires sanguins et les cellules. Il a en effet une composition ionique proche de celle du plasma sanguin.

Ces systèmes évolués permettent ainsi de surveiller les paramètres biochimiques souhaités de façon transcutanée, sans nécessité de percer régulièrement la peau et de prélever.

En particulier, il a été proposé un dispositif porté au poignet appelé GlucoWatch, mettant en oeuvre un phénomène appelé iontophorèse (ou ionophorèse) dans lequel un champ électrique permet « d'attirer » le liquide interstitiel à travers la peau jusqu'à un capteur sur la paroi du dispositif. Ce concept a cependant été abandonné rapidement car 6% seulement des patients supportaient la douleur d'extraction électrique. De surcroît les résultats des mesures étaient peu fiables.

Il a été proposé alternativement des sondes transcutanées prenant la forme d'un patch autocollant plaquant un « capteur-microaiguille » juste sous la peau, de sorte à mettre le capteur en communication fluidique permanente avec le liquide interstitiel, pour une surveillance continue. Certaines de ces sondes transcutanées de type patch comprennent des moyens de communication sans fil permettant de remonter les mesures sur le liquide interstitiel à un terminal mobile, pour un stockage et/ou un traitement des mesures (vérification de seuils et de variations, réalisation de statistiques, déclenchement d'alertes si nécessaire, *etc.*). On citera par exemple les systèmes sugarBEAT^{™} ou FreeStyle Libre.

Des capteurs pour système de surveillance corporelle comprenant des microaiguilles sont connus de US-2006/030812-A1, US-2018/362334-A1, CN-109303976-A, US-2018/338713-A1, US-2015/313527-A1, US-2018/161563-A1 et US-2019/143090-A1.

De nombreux systèmes proposés comprennent un réseau de microaiguilles. Cependant, il s'avère que le port prolongé et immobile d'un réseau de microaiguilles en contact avec la peau entraîne des irritations et favorise le développement de bactéries.

### EXPOSE DE L'INVENTION

Le but principal de l'invention est de remédier aux problèmes ainsi posés. La présente invention a en particulier pour but de proposer un capteur comprenant un réseau de microaiguilles dont le port par un utilisateur limite les irritations et/ou le développement de bactéries.

Ce but est atteint dans le cadre de la présente invention grâce à un capteur pour système de surveillance corporelle selon la revendication 1. L'invention est avantageusement complétée par les caractéristiques suivantes, prises individuellement ou en l'une quelconque de leurs combinaisons techniquement possibles :
- le canal présente une hauteur minimale dans la direction de l'axe central supérieure à 100 µm, et présentant une largeur minimale supérieure à 1000 µm dans une direction radiale à l'axe central, le canal formant une cavité dans laquelle est logée la microaiguille,
- le substrat comprend au moins un passage ouvert, et préférentiellement au moins deux passages ouverts, assurant une liaison entre le canal et l'extérieur de sorte à renouveler l'air entourant la base d'une ou plusieurs microaiguilles 20.,
- le substrat a la forme générale d'un anneau présentant un orifice central et le passage relie le canal de chaque aiguille à l'orifice central,
- le passage présente une hauteur minimale dans la direction de l'axe central supérieure à 100 µm, et présente une largeur minimale supérieure à 100 µm dans une direction radiale à l'axe central,
- le substrat support comprend une ouverture reliant au moins deux canaux, chaque canal entourant la base d'au moins une microaiguille,
- le substrat comprend au moins un passage ouvert commun à au plus deux cents microaiguilles et préférentiellement au plus huit microaiguilles,
- le capteur comprend au moins un passage reliant le canal à l'extérieur et comporte une barrière en matériau semi-perméable, soit imperméable à l'eau et perméable à l'air,
- le capteur comprend un passage reliant le canal à l'extérieur, ledit passage ayant une section adaptée pour empêcher l'entrée d'eau par répulsion capillaire.

Un autre aspect de l'invention est un système de surveillance corporelle, comprenant un capteur conforme à l'invention, et comprenant en outre un module configuré pour exploiter un signal électrique délivré par le capteur et fournir une information représentative d'un analyte.

De préférence ce système comprend en outre au moins une ouverture reliant au moins un canal à l'extérieur du système.

Un autre aspect de l'invention est un procédé de surveillance corporelle comprenant une étape dans laquelle on utilise un capteur conforme à l'invention.

### DESCRIPTION DES FIGURES

D'autres caractéristiques, buts et avantages de la présente invention apparaîtront à la lecture de la description détaillée qui va suivre, et au regard des dessins annexés, donnés à titre d'exemples non limitatifs et sur lesquels :
[Fig. 1] - la figure 1 est une vue en perspective d'un capteur comprenant quatre microaiguilles,
[Fig. 2] - la figure 2 est une vue en perspective selon un angle d'observation différent de la figure 1 d'un tel capteur,
[Fig. 3] - la figure 3 représente une vue face arrière d'un tel capteur,
[Fig. 4] - la figure 4 représente une vue latérale de ce capteur,
[Fig. 5] - la figure 5 représente une vue côté pointe du capteur,
[Fig. 6] - la figure 6 représente une vue latérale d'une microaiguille selon un mode de réalisation de l'invention,
[Fig. 7] - la figure 7 représente une vue schématique en perspective d'un système de surveillance corporelle incorporant un capteur conforme à la présente invention,
[Fig. 8] - la figure 8 représente une vue d'une capsule adaptée pour porter une pluralité de capteurs conforme à l'invention,
[Fig. 9] - la figure 9 représente une vue à échelle agrandie d'une partie de cette capsule,
[Fig. 10] - la figure 10 représente une vue à échelle agrandie d'une partie d'une capsule selon un mode de réalisation différent de l'invention,
[Fig. 11] - la figure 11 illustre schématiquement une coupe d'une microaiguille montée fixe sur un substrat formant un canal d'air qui entoure la base de la microaiguille.

### DEFINITION

On entend par « électrode » un dispositif conducteur permettant de capter les variations de potentiel électrique chez un organisme vivant. Une électrode comprend une borne comportant une extrémité de connexion et au moins une extrémité de détection par lesquelles un potentiel électrique ou un courant électrique est transmis, chaque extrémité de détection étant portée par une microaiguille. L'électrode peut ainsi comporter une extrémité de détection unique. Elle peut aussi comporter une pluralité d'extrémités de détection. Dans ce cas, on notera que l'électrode reste unique, quand bien même plusieurs extrémités de détection sont destinées à pénétrer dans le corps d'un organisme vivant.

### DESCRIPTION DETAILLEE DE L'INVENTION

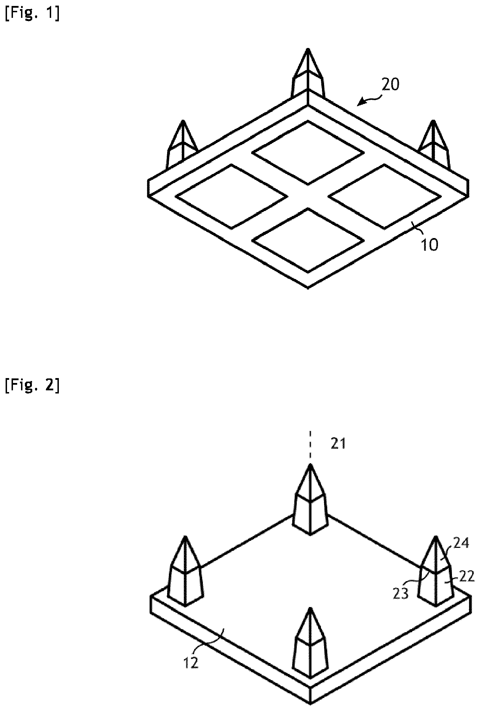

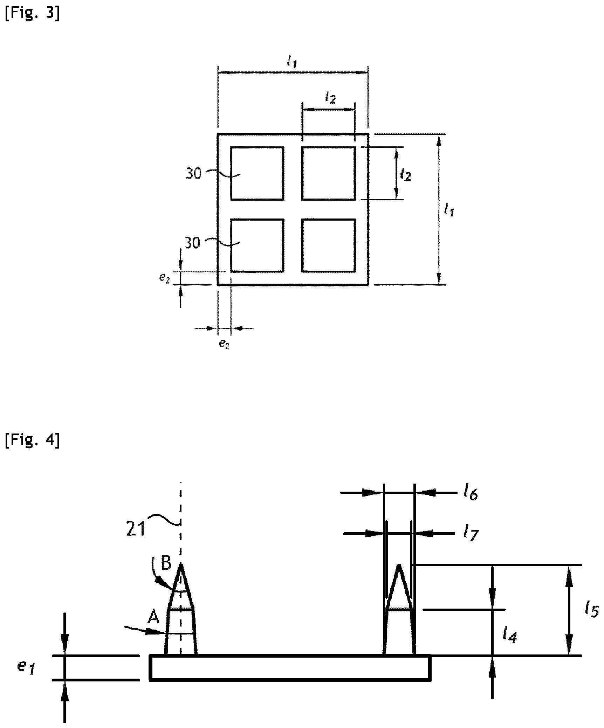

On va décrire le mode de réalisation particulier et non limitatif d'un capteur conforme à la présente invention tel qu'illustré sur les figures 1 à 6 annexées

### Microaiguille(s)

En référence aux figures 1 à 6, un capteur comprend une platine support 10 munie de quatre microaiguilles 20. Le contour de la platine peut faire l'objet de nombreuses variantes de réalisation. Selon la représentation donnée sur les figures 1 à 5, la platine 10 a un contour carré. Les quatre microaiguilles sont situées respectivement à proximité des angles de la platine 10. Les microaiguilles 20 s'étendent perpendiculairement au plan de base de la platine 10. En d'autres termes, l'axe central 21 de chaque microaiguille 20 s'étend perpendiculairement à la surface de base de la platine 10.

En référence aux figures 1 à 3, la face de la platine 10 opposée aux microaiguilles 20 comprend quatre plages 30 électriquement conductrices, chaque plage 30 étant reliée électriquement à la partie active 25 de chaque microaiguille 20. Les plages 30 électriquement conductrices permettent une continuité électrique d'une électrode quand elles sont reliées électriquement à une embase, par exemple de travail ou d'une contre-électrode, dans la microaiguille 20, et ce potentiellement de manière séparée de sorte que chaque électrode de travail soit indépendante des autres électrodes de travail. En pratique, ces plages 30 peuvent être venues de matière avec les microaiguilles 20 ou reliées électriquement aux microaiguilles 20 par tout moyen approprié à travers ou autour de la platine 10. La platine 10 support peut être réalisée en tout matériau approprié électriquement, par exemple électriquement isolant ou conducteur. De même, les microaiguilles 20 peuvent être formées en tout matériau approprié. Elles sont propres à véhiculer un signal électrique capté par la surface active 25. A titre préférentiel, les microaiguilles 20 peuvent être formées à base de polycarbonate ou de silicium. La ou les microaiguilles 20 sont préférentiellement pleines, c'est-à-dire dénuées de cavité. Ainsi, la fabrication de microaiguilles 20 adaptées à la mesure d'analyte est facilitée, tout en permettant une mesure électrochimique d'un analyte. La microaiguille peut comprendre majoritairement du silicium. Dans le cas du silicium, la microaiguille présente une couche externe de SiOz de protection non conductive, formée par oxydation du silicium en surface. Ainsi, la microaiguille peut ne pas comprendre de revêtement additionnel à la couche de SiO₂.

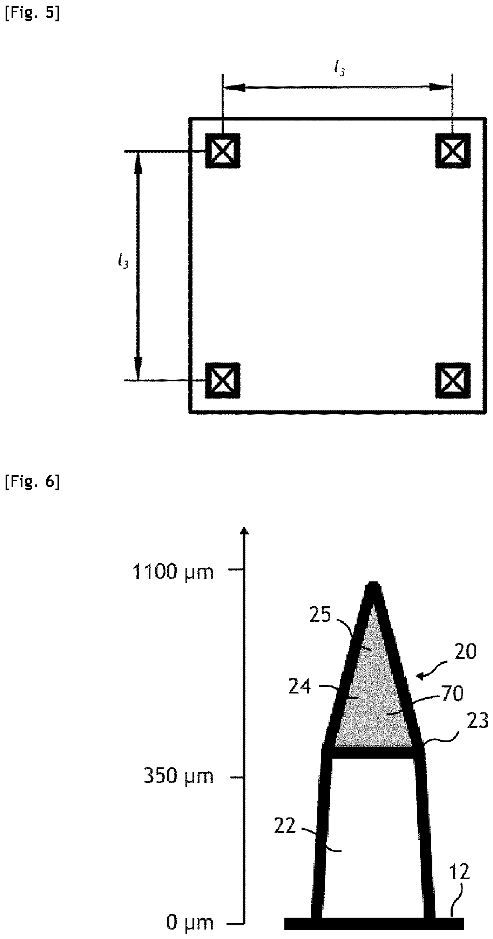

En référence à la figure 2 et à la figure 6, chaque microaiguille 20 comprend un fût de base 22 et un sommet en pointe 24. Le fût 22 peut préférentiellement s'amincir vers le sommet en pointe 24 de la microaiguille 20. Le sommet en pointe 24 présente une pente supérieure à celle du fût 22, c'est-à-dire qu'il forme un angle B avec l'axe central 21 supérieur à l'angle A formé entre fût de base 22 et l'axe central 21. La microaiguille 20 présente une cassure ou transition de pente 23 entre le fût de base 22 et le sommet en pointe. La transition de pente 23 peut être matérialisée par une arête.

Le fût de base 22 et le sommet en pointe 24 peuvent présenter une section carrée. Dans ce cas, le fût 22 de base est quadrangulaire et la pointe 24 est de type pyramidal. L'ensemble de la microaiguille 20 peut préférentiellement avoir la forme d'un obélisque.

En variante cependant, la microaiguille 20 peut présenter une section circulaire. Dans ce cas, le fût 22 de base a la forme d'un tronc de cône circulaire de révolution et la pointe 24 est formée d'une pointe conique de révolution.

De préférence, le sommet en pointe 24 s'étend exclusivement à une distance comprise entre 350 µm et 1100 µm de la base du fût de base 22 de la microaiguille, c'est-à-dire de la face 12 de la platine support 10, et préférentiellement entre 600 µm et 1000 µm de la base de la microaiguille et cette surface 12 de la platine support 10. On entend par « s'étend exclusivement à une distance comprise entre 350 µm et 1100 µm » que la partie du sommet en pointe 24 la plus proche de la base du fût de base est agencée à une distance supérieure à 350 µm de la base du fût de base 22, et que la partie la plus distante de la base du fût de base 22 est agencée à une distance inférieure à 1100 µm.

Par ailleurs, des essais ont montré que la surface de la partie active 25 de détection doit être comprise entre 0,04 et 0,9 mm². Par conséquent, lorsque la mesure est faite avec une microaiguille 20 unique, la partie active 25 de cette microaiguille 20 est comprise entre 0,04 et 0,9 mm². Lorsque la mesure est réalisée avec plusieurs microaiguilles 20, la surface de la partie active précitée, comprise entre 0,04 et 0,9 mm² s'entend de la surface totale active des microaiguilles considérées.

L'homme de l'art comprendra que la forme d'obélisque des microaiguilles 20, ou la forme d'un corps similaire circulaire de révolution mais présentant une rupture de pente entre le fût 22 de base et le sommet en pointe 24, permet de résoudre un problème posé par les microaiguilles 20 connues de l'état de l'art, à savoir minimiser le diamètre de pénétration dans la peau tout en maximisant la surface de la partie active 25 présente dans la partie de la peau comprise entre l'épiderme et les nerfs.

Les microaiguilles 20 conformes à la présente invention peuvent être réalisées à l'aide de tout procédé de microfabrication approprié.

La partie active 25 comprend une face électriquement conductrice, préférentiellement recouverte d'un revêtement faisant l'objet de diverses variantes selon le type de mesure recherché et le type d'analyte à mesurer. Pour une mesure de glycémie, la partie active 25 est pourvue d'un revêtement propre à mettre en oeuvre une réaction enzymatique avec le glucose. La partie active 25 peut également ne pas comprendre de revêtement spécifique à un analyte prédéterminé, par exemple dans le cas de la partie active 25 d'une contre-électrode ou d'une électrode de référence.

Par ailleurs, selon le mode de réalisation particulier représenté sur les figures 1 à 5, et à titre d'exemple non limitatif :
- la hauteur *l*₄ du fût de base 22 est de l'ordre de 380 µm,
- la hauteur totale *l*₅ de chaque microaiguille 20 est de l'ordre de 750 µm,
- la largeur à la base *l*₆ de chaque microaiguille 20 est de l'ordre de 0,25mm,
- la largeur *l*₇ de la microaiguille au niveau de la transition de pente 23 est de l'ordre de 0,2mm,
- l'angle A de convergence du fût de base 22 par rapport à l'axe central 21 est de l'ordre de 7°,
- l'angle B du sommet en pointe 24 par rapport à l'axe central 21 est de l'ordre 30° .

### Réseau de microaiguilles

La ou les microaiguilles 20 conformes à l'invention permettent de réduire le nombre de microaiguilles 20 d'une électrode de travail 70. Préférentiellement, une électrode de travail comprend entre une et sept, notamment entre une et cinq et préférentiellement entre une et trois parties actives 25 recouvrant chacune au moins une partie de la surface du sommet en pointe 24 d'une microaiguille 20 différente. Les systèmes connus de l'état de l'art ne permettent pas d'utiliser aussi peu de microaiguilles.

L'invention permettant de réduire drastiquement le nombre de microaiguilles 20 nécessaires à la mesure par rapport aux systèmes connus de l'état de l'art, il est possible, pour une surface de capteur donnée, de minimiser la densité de microaiguilles. Chaque paire de microaiguilles 20 adjacentes est préférentiellement séparée d'une distance entre les pointes des sommets en pointe 24 d'au moins 1mm et préférentiellement d'au moins 1,5 mm, voir le cas échéant d'au moins 1,8mm. Ceci a pour effet d'éviter une déformation homogène de la peau lors de la mise en contact d'un réseau de microaiguilles 20 avec la peau, connue dans d'autres domaines techniques sous le nom d'effet fakir, et au contraire de favoriser une déformation de la peau localisée autour de chacune des microaiguilles. Ainsi, la douleur entraînée par la pénétration d'aiguilles dans la peau peut être significativement diminuée, voire supprimée et la pénétration se fait très naturellement, les aiguilles étant de fait devenues indépendantes mécaniquement.

Par ailleurs, selon le mode de réalisation particulier représenté sur les figures 1 à 5, et à titre d'exemple non limitatif, l'entraxe *l*₃ entre chaque paire de microaiguilles 20 est de l'ordre de 1,5 mm.

### Electrodes

Le capteur est préférentiellement adapté pour mesurer la présence ou la concentration d'un analyte par électrochimie. Un capteur peut comprendre une électrode de travail 70, adaptée pour évaluer la présence d'un analyte dans le corps d'un utilisateur. L'électrode de travail 70 comprend au moins une première extrémité reliée électriquement à un module configuré pour exploiter le signal électrique de l'électrode de travail 70, et au moins une deuxième extrémité formée par la partie active 25. Elle peut également comprendre une pluralité de deuxièmes extrémités. La partie active 25 de la microaiguille 20 recouvre au moins une partie de la surface du sommet en pointe 24 et préférentiellement la totalité de la surface du sommet en pointe 24. A cet effet, la partie active 25, au niveau du sommet en pointe 24, est revêtue de tout revêtement approprié pour la mesure souhaitée, typiquement un revêtement adapté à détecter la glycémie par électrochimie.

Le capteur peut comprendre une contre-électrode. La contre-électrode peut comprendre une première extrémité destinée à être reliée électriquement à un module configuré pour exploiter un signal électrique, et au moins une autre extrémité permettant d'exploiter un signal électrique dans le corps de l'utilisateur. L'autre extrémité de la contre-électrode peut recouvrir une microaiguille de contre électrode, par exemple une microaiguille conforme à l'invention. Cependant, la contre-électrode n'a pas les mêmes prérequis de surface active que l'électrode de travail. Ainsi, le sommet en pointe de la contre-électrode peut s'étendre exclusivement à une distance comprise entre 100 µm et 1100 µm du fût de base de la microaiguille. En variante, l'autre extrémité de la contre-électrode peut recouvrir toute la surface de la microaiguille de contre-électrode.

Dans le cas d'un capteur conforme à un mode de réalisation de l'invention comprenant plusieurs électrodes de travail, chaque électrode de travail peut être adaptée à détecter le même analyte qu'une autre électrode de travail, ou être adaptée à détecter un analyte différent d'une autre électrode de travail.

Dans le cas où plusieurs électrodes de travail sont adaptées à détecter le même analyte, chaque électrode de travail peut comprendre une partie active 25 comprenant le même type de revêtement. Ainsi, il peut être possible de mettre en oeuvre des mesures indépendantes pour le même analyte, et ainsi d'obtenir une meilleure précision de mesure de l'analyte. Chaque électrode de travail peut également comprendre des parties actives 25 différentes, comprenant des revêtements différents, mais adaptés à détecter le même analyte. La concentration de l'analyte peut ainsi être détectée avec plus de précision qu'en utilisant un seul revêtement pour la partie active 25.

Chaque électrode peut également être adaptée à détecter des analytes différents. Ainsi, il est possible de surveiller plusieurs pathologies avec le même système de surveillance.

### Platine support

Comme vu précédemment, la ou les microaiguilles 20 peuvent être agencées sur une platine support 10. En référence aux figures 1 à 4, l'épaisseur e₁ de la platine support est avantageusement comprise entre 0,1mm et 1 mm, et préférentiellement de l'ordre de 0,2 mm. Les dimensions des microaiguilles 20 peuvent faire l'objet de nombreuses variantes de réalisation. Il en est de même pour la platine support 10. A titre d'exemple non limitatif en référence au mode de réalisation particulier représenté sur les figures 1 à 5, la platine support 10 présente des côtés ayant une largeur *l*₁ inférieure à 10mm, avantageusement inférieure à 3mm, par exemple de l'ordre de 2,3mm. Les plages électriquement conductrices 30 sont des plages par exemple carrées ayant un côté *l*₂ de l'ordre de 0,8mm. Ces plages 30 peuvent être situées à une distance e₂ de l'ordre de 0,2 mm des bords de la platine support.

Les platines supports 10 peuvent elles-mêmes faire l'objet de différentes variantes de réalisation. Certaines platines supports 10 peuvent être adaptée à supporter par exemple quatre microaiguilles tandis que d'autres platines supports 10 peuvent être adaptées à supporter seulement deux microaiguilles 20.

### Système de mesure

La présente invention peut permettre de mesurer de manière indépendante le niveau de glycémie à l'aide d'une pluralité d'électrode de travail 70. Ceci présente un avantage indéniable par rapport à l'état de l'art selon lequel une telle mesure indépendante à l'aide d'une électrode de travail comprenant une microaiguille unique n'était pas envisageable car le signal de mesure était trop bruité en utilisant une seule microaiguille. Ainsi, le capteur comprend préférentiellement plusieurs électrodes de travail 70, le système de mesure étant adapté à mesurer individuellement le potentiel électrique de chacune des électrodes de travail 70.

La mesure du potentiel de chacune des électrodes de travail 70 peut être multiplexée. Dans le cadre de la présente invention, il est proposé avantageusement des moyens permettant de rejeter les valeurs de mesure minimales ou maximales du potentiel associé à un ensemble de mesures, ce qui était impossible en utilisant les capteurs de l'art antérieur.

Le capteur conforme à la présente invention peut être mis en oeuvre dans différents types de système de surveillance corporelle.

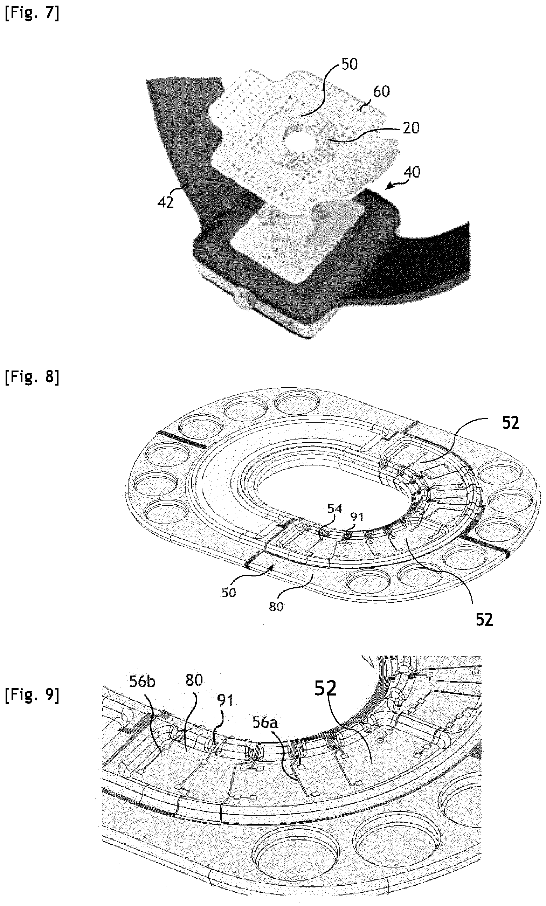

De préférence, le capteur conforme à l'invention est mis en oeuvre dans un système de surveillance du type illustré sur les figures 7 à 10 annexées.

Un tel système comprend un boîtier 40 en forme de boîtier de montre comportant un bracelet 42 adapté pour entourer le poignet d'un individu. Le boîtier 40 loge un module configuré pour exploiter le signal électrique délivré par chaque microaiguille 20 et fournir une information représentative d'une grandeur physique du fluide, typiquement d'un taux de glycémie.

Comme on l'a évoqué précédemment, le système de surveillance corporelle utilisé préférentiellement selon l'invention comprend une capsule 50 comprenant au moins un capteur du type précité, et de préférence une pluralité de capteurs comme on le décrira plus en détail par la suite.

Le système de surveillance corporelle conforme à l'invention comprend par ailleurs un patch 60 auquel est liée la capsule 50, le patch 60 étant lui-même pourvu d'un adhésif permettant de faire adhérer l'ensemble patch et capsule 50 sur la peau d'un individu.

Comme on l'a représenté sur la figure 8 et en partie sur la figure 9, la capsule 50 a de préférence la forme générale d'un anneau comportant une pluralité de logements en creux 52 adaptés pour recevoir chacun respectivement la platine support 10 d'un capteur précité.

En référence aux figures 7 et 8, la capsule 50 peut comprendre des plages électriquement conductrices 54 destinées à être placées en regard des plages électriquement conductrices 30 prévues sur la platine support 10, pour assurer une liaison électrique entre les microaiguilles 20 et le module prévu dans le boîtier 40 pour exploiter le signal électrique ainsi prélevé. Les plages 54 sont elles-mêmes interconnectées avec le module précité par des pistes électriquement conductrices 56a.

Comme on le voit à l'examen de la figure 9, certaines des plages 54 peuvent être reliées individuellement au module de traitement précité par des pistes 56a respectives tandis que d'autres plages 54 peuvent être reliées au module de traitement par des pistes communes 56b.

### Limitation des irritations

En référence aux figures 8, 9, 10 et 11, le capteur comprend un substrat sur lequel la ou les microaiguilles sont montées fixes. Par substrat, on entend par exemple l'ensemble d'une platine support 10 et d'une embase support 80, ou la surface d'une capsule 50. Le substrat, ou au moins une partie du substrat, est destinée à venir en contact avec la peau de l'utilisateur. Le substrat comprend un canal 90, ouvert, qui entoure la base de la ou des microaiguilles 20. Préférentiellement, une paroi d'une ou plusieurs microaiguilles forme(nt) une paroi du canal 90. On entend par canal « ouvert » que l'une des parois du canal 90, ou une partie des parois du canal 90 peut être manquante, le canal 90 étant adapté à ce que la peau d'un utilisateur, quand elle est en contact avec le substrat, vienne former la paroi manquante du canal 90. Ainsi, quand la peau est en contact avec le substrat, un canal 90 d'air entoure la ou les microaiguilles 20. Cela permet de garder l'ouverture de la peau entraînée par la pénétration de la microaiguille en contact avec l'air. Ainsi, les irritations sont évitées.

La peau présente une souplesse qui lui permet de s'adapter à différents types de formes non planes. Le canal présente une hauteur minimale h dans la direction de l'axe central 21 d'une microaiguille supérieure à 20 µm. Ainsi, la peau ne s'enfonce pas dans le canal 90 pour des pressions élevées exercées par la peau sur le substrat, et le canal 90 ne peut être bouché par la peau. Avantageusement la hauteur minimale h du canal est supérieure à 100 µm.

Le canal présente préférentiellement une largeur minimale dans une direction radiale à l'axe central 21 supérieure à 200 µm et préférentiellement à 1000 µm. Cela permet de garder un réservoir d'air suffisamment élevé pour la cicatrisation de la peau autour de la microaiguille 20.

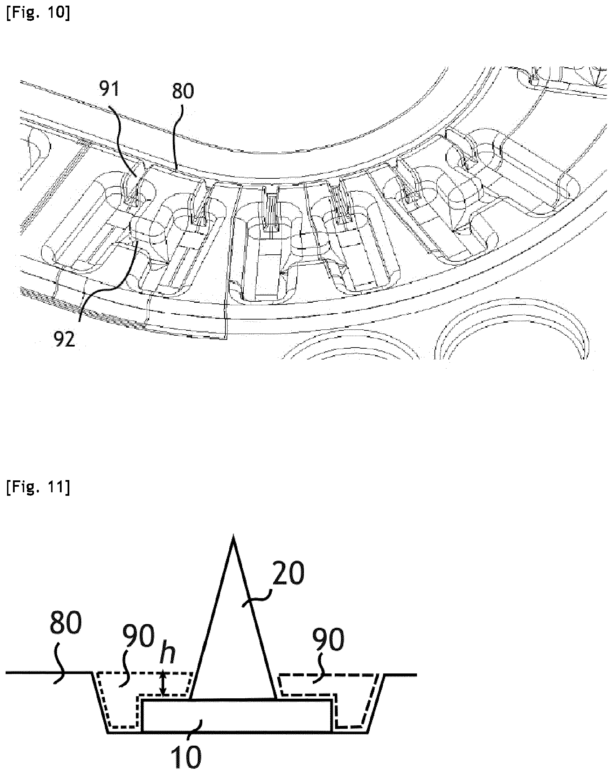

Le canal 90 peut être formé par de nombreuses géométries. Préférentiellement, et en référence à la figure 11, le substrat peut présenter une cavité dans laquelle est logée la microaiguille 20, formant le canal 90 ouvert autour de la base de la microaiguille 20.

Le canal 90 peut être relié à l'extérieur du capteur et/ou du système par un passage 91. Le passage 91 peut être défini par le substrat, comme le canal 90. Le passage 91 peut être un passage 91 ouvert, c'est-à-dire que l'une des parois du passage 91, ou une partie des parois du passage 91 peut être manquante, le passage 91 étant adapté à ce que la peau d'un utilisateur, quand elle est en contact avec le substrat, vienne former la paroi manquante du passage 91. Ainsi, l'air entourant la base d'une ou plusieurs microaiguilles 20 peut être renouvelé *via* le passage 91, ce qui permet par exemple de réguler le taux d'humidité dans le canal 90 et d'éviter les irritations de la peau. Le passage 91 peut présenter une hauteur minimale dans la direction de l'axe central 21 supérieure à 100 µm, et présente une largeur minimale supérieure à 100 µm dans une direction transversale à l'axe central 21. Ainsi, il est possible d'assurer un débit d'air suffisant en direction du canal 90 ou en direction de l'extérieur, de manière à éviter les irritations de la peau. Par « relié », on entend que le passage 91 présente une sortie fluidique qui définit également une entrée fluidique du canal 90, le passage 91 et le canal 90 étant reliés de manière directe.

Le substrat peut avoir préférentiellement la forme générale d'un anneau présentant un orifice central. Le passage 91 peut relier le canal 90 de chaque aiguille à l'orifice central. Ainsi, il est possible de protéger l'intrusion d'éléments nocifs à la cicatrisation de la peau directement de l'extérieur du capteur ou du dispositif.

Le passage présente une largeur d'au moins 100 µm et préférentiellement d'au moins 300 µm dans une direction transversale à l'axe central 21. Ainsi, le renouvellement de l'air provenant de l'extérieur du capteur peut être assuré sans risquer de boucher le passage 91.

Le capteur peut également comprendre au moins une ouverture 92, permettant de relier au moins deux canaux 90. Ainsi, si l'un des canaux 90 n'est pas relié par un passage 91, l'air entourant la ou les microaiguilles 20 peut tout de même être renouvelé via l'ouverture 92. Un passage 91 peut ainsi être préférentiellement commun à au plus deux cents microaiguilles, avantageusement au plus seize microaiguilles et préférentiellement au plus huit microaiguilles. Au-delà, l'approvisionnement d'air peut être plus difficile.

Le passage 91 peut préférentiellement comprendre une barrière en matériau semi-perméable, c'est-à-dire imperméable à l'eau et perméable à l'air. Ainsi, si le capteur et/ou le système est fortuitement mis en contact avec de l'eau, l'introduction d'eau autour des aiguilles peut être évitée. En variante, il est possible de définir une largeur du ou des passage 91 assez petite pour éviter l'introduction d'eau par répulsion capillaire. La largeur du passage 91 peut ainsi être inférieure à 500 µm et préférentiellement inférieure à 400 µm.

### Mise en oeuvre d'une mesure d'analyte corporel

Comme on l'a indiqué précédemment, la présente invention concerne également un procédé de surveillance corporelle à l'aide d'un capteur comportant une microaiguille du type précité.

Le procédé de surveillance comprend une étape de mesure d'analyte corporel à l'aide d'une microaiguille 20 conforme à un mode de réalisation de l'invention. De par les caractéristiques de la microaiguille 20, le capteur peut comprendre une pluralité d'électrodes de travail. La mesure peut par exemple être mise en oeuvre en polarisant la ou les électrodes de travail et la ou les contre-électrodes à un potentiel électrique adapté pour entraîner une réaction d'oxydo-réduction impliquant l'analyte à mesurer.

L'étape de mesure peut être préférentiellement mise en oeuvre au moins à l'aide de deux électrodes de travail différentes. L'étape de mesure peut être par exemple mise en oeuvre indépendamment, successivement sur chacune des électrodes de travail 70, ou en parallèle sur chacune des électrodes de travail 70. Ainsi, la concentration de l'électrolyte peut être analysée de manière plus précise qu'avec un système comprenant par exemple une seule électrode de travail présentant plusieurs extrémités sous forme de microaiguilles.

Un autre aspect de l'invention est un procédé de mesure d'analyte corporel comprenant une étape de pénétration des microaiguilles d'un capteur conforme à l'invention dans la peau d'un utilisateur. Ainsi, les aiguilles du capteur peuvent être introduites dans la peau sans applicateur, de par l'espacement des microaiguilles 20. Une force basse en comparaison de la force procurée par un applicateur peut être utilisée pour la pénétration des microaiguilles. Préférentiellement, une force inférieure à 50 newtons, et préférentiellement inférieure à 30 newtons, peut être utilisées pour la pénétration des microaiguilles 20. Ainsi, la pénétration des microaiguilles peut être mise en oeuvre avec la main, ou préférentiellement avec des moyens d'attache mécanique du système, par exemple un bracelet.

## Revendications

1. Capteur pour système de surveillance corporelle comprenant au moins un substrat destiné à venir en contact avec la peau, et au moins une microaiguille, la microaiguille (20) étant montée fixe sur le substrat, le substrat comprenant un canal (90) ouvert qui entoure la base de la microaiguille, la microaiguille présentant un axe central (21) de symétrie, et le canal présentant une hauteur minimale (h) dans la direction de l'axe central (21) supérieure à 20 µm, et présentant une largeur minimale supérieure à 200 µm dans une direction radiale à l'axe central (21), le canal formant une cavité dans laquelle est logée la microaiguille (20), le substrat comprenant au moins un passage (91) ouvert, défini par le substrat et assurant une liaison entre le canal (90) et l'extérieur de sorte à renouveler l'air entourant la base d'une ou plusieurs microaiguilles 20.

2. Capteur selon la revendication 1, dans lequel le canal présente une hauteur minimale (*h)* dans la direction de l'axe central (21) supérieure à 100 µm, et présentant une largeur minimale supérieure à 1000 µm dans une direction radiale à l'axe central (21), le canal formant une cavité dans laquelle est logée la microaiguille (20).

3. Capteur selon l'une des revendications 1 ou 2, **caractérisé par le fait que** le substrat a la forme générale d'un anneau présentant un orifice central et le passage (91) relie le canal (90) de chaque aiguille à l'orifice central.

4. Capteur selon la revendication 3, dans lequel le passage (91) présente une hauteur minimale dans la direction de l'axe central (21) supérieure à 100 µm, et présente une largeur minimale supérieure à 100 µm dans une direction transversale à l'axe central (21),

5. Capteur selon l'une des revendications 1 à 4, **caractérisé par le fait que** le substrat support comprend une ouverture (92) reliant au moins deux canaux (91), chaque canal entourant la base d'au moins une microaiguille (20).

6. Capteur selon l'une des revendications 1 à 5, **caractérisé par le fait que** le substrat comprend au moins un passage (91) ouvert commun à au plus deux cents microaiguilles (20), avantageusement au plus seize microaiguilles et préférentiellement au plus huit microaiguilles.

7. Capteur selon l'une des revendications 1 à 6, **caractérisé par le fait qu'**il comprend au moins un passage (91) reliant le canal à l'extérieur et comportant une barrière en matériau semi-perméable, soit imperméable à l'eau et perméable à l'air.

8. Capteur selon l'une des revendications 1 à 7, **caractérisé par le fait qu'**il comprend un passage (91) reliant le canal (90) à l'extérieur, ledit passage (91) ayant une section adaptée pour empêcher l'entrée d'eau par répulsion capillaire.

9. Système de surveillance corporelle, comprenant un capteur conforme à l'une des revendications 1 à 8, et comprenant en outre un module configuré pour exploiter un signal électrique délivré par le capteur et fournir une information représentative d'un analyte.

10. Système selon la revendication 9, comprenant en outre au moins une ouverture reliant au moins un canal (90) à l'extérieur du système.

11. Procédé de surveillance corporelle comprenant une étape dans laquelle on utilise un capteur pour système de surveillance corporelle conforme à l'une des revendication 1 à 8.

## Patentansprüche

1. Sensor für ein Körperüberwachungssystem, umfassend mindestens ein Substrat, das dafür bestimmt ist, mit der Haut in Kontakt zu kommen, und mindestens eine Mikronadel, wobei die Mikronadel (20) fest auf dem Substrat montiert ist,
wobei das Substrat einen offenen Kanal (90) umfasst, der die Basis der Mikronadel umgibt, wobei die Mikronadel eine zentrale Symmetrieachse (21) aufweist und der Kanal eine Mindesthöhe (h) in Richtung der zentralen Achse (21) von mehr als 20 µm und eine Mindestbreite von mehr als 200 µm in einer zu der zentralen Achse (21) radialen Richtung aufweist, wobei der Kanal einen Hohlraum bildet, in dem die Mikronadel (20) untergebracht ist, wobei das Substrat mindestens einen offenen Durchgang (91) umfasst, der durch das Substrat definiert ist und eine Verbindung zwischen dem Kanal (90) und der Außenseite herstellt, so dass die Luft, die die Basis einer oder mehrerer Mikronadeln (20) umgibt, erneuert wird.

2. Sensor nach Anspruch 1, wobei der Kanal eine Mindesthöhe (h) in Richtung der zentralen Achse (21) von mehr als 100 µm und eine Mindestbreite von mehr als 1000 µm in einer zu der zentralen Achse (21) radialen Richtung aufweist, wobei der Kanal einen Hohlraum bildet, in dem die Mikronadel (20) untergebracht ist.

3. Sensor nach einem der Ansprüche 1 oder 2, **dadurch gekennzeichnet, dass** das Substrat die allgemeine Form eines Rings mit einer zentralen Öffnung hat und der Durchgang (91) den Kanal (90) jeder Nadel mit der zentralen Öffnung verbindet.

4. Sensor nach Anspruch 3, wobei der Durchgang (91) eine Mindesthöhe in Richtung der zentralen Achse (21) von mehr als 100 µm und eine Mindestbreite von mehr als 100 µm in einer Richtung quer zu der zentralen Achse (21) aufweist.

5. Sensor nach einem der Ansprüche 1 bis 4, **dadurch gekennzeichnet, dass** das Trägersubstrat eine Öffnung (92) aufweist, die mindestens zwei Kanäle (91) verbindet, wobei jeder Kanal die Basis mindestens einer Mikronadel (20) umgibt.

6. Sensor nach einem der Ansprüche 1 bis 5, **dadurch gekennzeichnet, dass** das Substrat mindestens einen offenen Durchgang (91) aufweist, der höchstens zweihundert Mikronadeln (20), vorteilhafterweise höchstens sechzehn Mikronadeln und vorzugsweise höchstens acht Mikronadeln gemeinsam ist.

7. Sensor nach einem der Ansprüche 1 bis 6, **dadurch gekennzeichnet, dass** er mindestens einen Durchgang (91) umfasst, der den Kanal mit der Außenseite verbindet und eine Barriere aus semipermeablem Material aufweist, das wasserundurchlässig und luftdurchlässig ist.

8. Sensor nach einem der Ansprüche 1 bis 7, **dadurch gekennzeichnet, dass** er einen Durchgang (91) umfasst, der den Kanal (90) mit der Außenseite verbindet, wobei der Durchgang (91) einen Querschnitt aufweist, der geeignet ist, den Eintritt von Wasser durch Kapillarabstoßung zu verhindern.

9. Körperüberwachungssystem, das einen Sensor nach einem der Ansprüche 1 bis 8 umfasst und ferner ein Modul umfasst, das so konfiguriert ist, dass es ein von dem Sensor geliefertes elektrisches Signal auswertet und eine für einen Analyten repräsentative Information liefert.

10. System nach Anspruch 9, das ferner mindestens eine Öffnung umfasst, die mindestens einen Kanal (90) mit der Außenseite des Systems verbindet.

11. Körperüberwachungsverfahren umfassend einen Schritt, bei dem ein Sensor für ein Körperüberwachungssystem nach einem der Ansprüche 1 bis 8 verwendet wird.

## Claims

1. Sensor for body monitoring system comprising at least one substrate intended to come into contact with the skin, and at least one microneedle, the microneedle (20) being mounted fixedly on the substrate, the substrate comprising an open channel (90) which surrounds the base of the microneedle, the microneedle having a central axis (21) of symmetry, and the channel having a minimum height (h) in the direction of the central axis (21) greater than 20 µm, and having a minimum width greater than 200 µm in a direction radial to the central axis (21), the channel forming a cavity in which the microneedle (20) is housed, the substrate comprising at least one open passage (91) defined by the substrate and providing a connection between the channel (90) and the outside so as to renew the air surrounding the base of one or more microneedles (20).

2. The sensor according to claim 1, wherein the channel has a minimum height (*h)* in the direction of the central axis (21) of greater than 100 µm, and has a minimum width of greater than 1000 µm in a direction radial to the central axis (21), the channel forming a cavity in which the microneedle (20) is housed.

3. The sensor according to one of claims 1 or 2, **characterized in that** the substrate has the general shape of a ring with a central orifice and the passage (91) connects the channel (90) of each needle to the central orifice.

4. The sensor as claimed in claim 3, wherein the passage (91) has a minimum height in the direction of the central axis (21) greater than 100 µm and has a minimum width greater than 100 µm in a direction transverse to the central axis (21).

5. The sensor according to one of claims 1 to 4, **characterized in that** the support substrate comprises an opening (92) connecting at least two channels (91), each channel surrounding the base of at least one microneedle (20).

6. The sensor according to one of claims 1 to 5, **characterized in that** the substrate comprises at least one open passage (91) common to at most two hundred microneedles (20), advantageously at most sixteen microneedles and preferably at most eight microneedles.

7. The sensor according to one of claims 1 to 6, **characterized in that** it comprises at least one passage (91) connecting the channel to the outside and comprising a barrier of semi-permeable material, i.e. impermeable to water and permeable to air.

8. The sensor according to one of claims 1 to 7, **characterized in that** it comprises a passage (91) connecting the channel (90) to the outside, the said passage (91) having a cross-section adapted to prevent the entry of water by capillary repulsion.

9. Body monitoring system, comprising a sensor according to one of claims 1 to 8, and further comprising a module configured to exploit an electrical signal delivered by the sensor and to provide information representative of an analyte.

10. The system according to claim 9, further comprising at least one opening connecting at least one channel (90) to the outside of the system.

11. A method of body monitoring comprising a step in which a sensor for a body monitoring system according to any one of claims 1 to 8 is used.
